# EUROPEAN PATENT APPLICATION

(11) **EP 2 436 772 A1**
(43) Date of publication of application: **04.04.2012**
(21) Application number: 10183799.5
(22) Date of filing: 30.09.2010
(51) Int. Cl.: C12P 7/18

(54) **Method for the production of erythritol**

(71) Applicant: Annikki GmbH, 8020 Graz (AT)
(72) Inventor: Mach-Aigner, Astrid, A-3011, Untertullnerbach (AT); Mach, Robert, A-3011, Untertullnerbach (AT)
(74) Representative: Bachinger-Fuchs, Eva-Maria

(57) **Abstract**

The invention relates to a method for the production of erythritol, in which erythrose is reduced to erythritol with a NADPH-specific erythrose reductase, wherein an erythrose reductase from the group of saprophytes selected from the strains of *Hypocrea, Gibberella, Aspergillus* and *Penicillium* is used for reduction. Hereby it is possible to produce erythritol in high yields.

## Description

The invention relates to a method for the production of erythritol, wherein erythrose is reduced to erythritol with a NADPH-specific erythrose reductase.

Polyols are carbohydrates whose carbonyl group (aldehyde or ketone) has been reduced to a primary or secondary hydroxyl group. These compounds are used in a variety of applications ro provide functional foods and nutraceuticals with value-added properties. Typical commercially available polyols include sorbitol, mannitol, xylitol, lactitol, maltitol, and erythritol.

Erythritol is a naturally occurring substance that is widely distributed in nature. It occurs as a metabolite or storage compound in seaweeds and fungi and is also a component of a number of common fruits, such as melons, grapes and pears. It occurs frequently in fermented foods (soy sauce), drinks (wines and beers), and in processed vegetables such as miso bean paste.

Erythritol has at least 60% to 70% of the sweetness of sucrose. Eythritol can be safely used as a noncariogenic sweetener in foods as it cannot be fermented by the bacteria that cause dental caries. Moreover, it is low in calories, it lacks the laxative properties that other sweeteners have and it is also suitable for diabetics. It has a positive heat of solution (endotherm) when dissolved in water providing a strong cooling effect.

Erythritol can be synthesized, for example, from dialdehyde starch by a high-temperature chemical reaction in the presence of a nickel catalyst. This process has, however, not been industrialized because of its low efficiency. Large-scale production of erythritol uses fermentative processes with pure glucose, sucrose, and glucose from chemically and enzymatically hydrolyzed wheat and corn starches. Furthermore, it can be produced by microbial methods that utilize osmophilic yeasts and some bacteria (Ryu, Y. W. et al., J. Ind. Microbiol. Biotechnol. 25: 100-103, 2000; Takafumi Kasumi, JARQ 29, 49-55, 1995).

The erythritol found in yeast and fungus species is produced via the so called pentose phosphate pathway. Hereby, erythritol is synthesized from D-erythrose-4-phosphate through dephosphorylation and subsequent reduction of erythrose. In the final step of this pathway erythrose reductase (ER) is playing a key role as catalyst.

Several erythrose reductases have been identified and characterized to date, e.g., erythrose reductase from *Candida magnoliae* (Lee et al., Appl. Environ. Microbiol. 69: 3710-3718, 2003) and from *Torula corallina* (Lee et al., Appl. Environ. Microbiol. 68: 4534-4538, 2002).

So far, research erfforts have focused on the discovery and selection of microorganisms capable of synthesizing erythritol in high yields, as well as on the optimization of fermentation processes (Moon et al., Appl. Microbiol. Biotechnol. 86: 1017-1025, 2010). The U.S. Patent 6,287,830 discloses a mutant strain of *Candida magnoliae*, which provides high yiels of erythritol under suitable conditions of culture.

The invention has as its object to provide a method which enables production of erythritol in high yields. Furthermore, it should enable the cost-efficient use of renewable biomass as the energy source for microbial production.

This object is achieved with a method of the initially mentioned kind, in that an erythrose reductase from the group of saprophytes selected from the strains of *Hypocrea*, *Gibberella*, *Aspergillus* and *Penicillium* is used for reduction.

It has been found that the said saprophytic fungus strains code for an enzyme which has erythrose reductase activity and, thus, reduces erythrose to erythritol in the presence of NADPH.

Preferably, in the method according to the invention, an erythrose reductase selected from the group of strains consisting of *Hypocrea jecorina (Trichoderma reesei), Gibberella zeae, Aspergillus niger, Aspergillus nidulans, Aspergillus oryzae* and *Penicillium chrysogenum* is used for reduction.

According to a preferred embodiment of the invention, an erythrose reductase from *Trichoderma reesei* is used for reduction. In particular, an erythrose reductase which is encoded by nucleic acid sequence SEQ ID NO:1 is used at this.

It is also preferred that a protein having a sequence SEQ ID NO:2, which is produced by *Trichoderma reesei,* is used for reduction.

The sequence identities of the proteins with erythrose reductase activity produced by the other saprophytes used according to the present invention are 69% for *Gibberella zeae,* 65% for *Aspergillus niger,* 66% for *Aspergillus nidulans,* 62% for *Aspergillus oryzae,* and 62% for *Penicillium chrysogenum*, based on the protein from *Trichoderma reesei* (SEQ ID NO:2). A preferred embodiment of the invention is characterized in that it is carried out as a whole cell process.

It is particularly preferred to use a recombinant fungus strain which overexpresses the erythrose reductase.

The saprophytes which are used according to the present invention advantageously differ from other microorganisms in that erythritol has not to be produced (indirectly) by intensive glucose feeding (40%) but in that the required sugar compound which is needed as the energy source (D-xylose in the present case) is provided via the saprophytic activity of the fungi. As is generally known, saprophytes are organisms that do not produce their nutrients by photosynthesis but derive them from decaying organic matter. Favourably, lignocellulosic biomass such as straw can thus be utilized because the saprophytes are provided with a native degradation pathway in regard to such material, resulting in xylose which continues into the pentose phosphate pathway leading to erythrose. In this way, it is feasible to produce erythritol from low-cost renewable biomass.

A further preferred embodiment of the invention is characterized in that overexpression of a erythrose reductase-coding gene of the recombinant strain is controlled by a promotor which is induced by xylose. Hereby, the production of erythritol can be increased in a simple manner.

The invention is illustrated and explained in more detail by way of the following examples.

### Examples:

### Microbial strains and growth conditions:

*E. coli* BL21 (DE3) pLysS and JM109 (both from Promega, Wisconsin, US) were used as recipient strains for plasmid transformation and maintained on LB/amp media containing 1 % (w/v) peptone, 1 % (w/v) NaCl, 0.5 % (w/v) yeast extract and ampicillin (100 µg/mL).

### PCR of the err1 structural gene:

An iCycler (Bio-Rad, Herkules, US) was used to run 30 cycles of 20 s 95 °C, 20 s 59 °C and 1 min 72 °C. We applied a 50 µL reaction comprising 5 x Flexi buffer (Promega), 2.5 mM MgCl, 6.25 µM primer forward and reverse, 0.2 mM dNTPs, 1 U GoTaq Polymerase (Promega) and *H. jecorina* cDNA as a template. Primers that have been used are err1_SalITf (5'-ATAGTCGACTATGTCTTCCGGAAGGACC-3') and err1_NotIr (5'-TATGCGGCCGCTTACAGCTTGATGACAGCAGTG-3'), introducing a *Sal*I and a *Not*I restriction enzyme site at the 5'- or 3'-end of the amplicon, respectively. The obtained 996 bp-fragment was subcloned into the pJET/1.2 vector (Fermentas, St. Leon-Rot, Germany) using the CloneJET PCR Cloning Kit (Fermentas) and *E. coli* JM109 thermo-competent cells (Promega) according to the manufacturers instructions to create pARMA2T. Via the introduced restriction enzyme sites *Sal*I and *Not*I *err1* was released from pARMA2T and further cloned into the expression vector pGEX-4T-2 (GE Healthcare) to create pARMA2. The insertion of the *err1* structural gene without mutations was confirmed by sequencing of the vector insert (MWG, Ebersberg, Germany).

### Transformation of E. coli:

The transformation of pARMA2T or pARMA2 in *E. coli* JM109 or BL21 (DE3) pLysS cells, respectively, was carried out according to a standard protocol from Promega). Therefore, 10 µl-ligation mixture or 0.1 µg (1 µl) of the pARMA2 plasmid were provided in one reaction tube and 100 µl of *E. coli* JM109 or BL21 (DE3) pLysS competent cells, respectively, were mixed and put on ice for 20 min. The uptake of plasmid-DNA was initiated by heat shock. Therefor, the tube was placed on the thermo block at 42 °C for 1.15 - 1.30 min. Then the tube was quickly put on ice for 2 min and 900 µl SOC medium (2 % (w/v) peptones, 0.5 % (w/v) yeast extract, 1M NaCl, 1M KCl, 1M MgCl2 or MgSO4 and 2M glucose) was added. For regeneration, the tube was placed on the thermo block at 37 °C for 1 h with gentle shaking. Thereafter, cells were harvested by centrifugation (5000 g, 2 min) and plated on LB/amp medium.

### Isolation of plasmid-DNA out of bacterial colonies:

Selected *E. coli* colonies, putatively containing pARMA2T or pARMA2, were cultivated in 4 mL LB/amp media at 37 °C over night. Cells were harvested by centrifugation at room temperature and plasmid-DNA preparation was performed by precipitating genomic DNA, cell debris and proteins as follows: After the cell pellet of each colony was properly mixed with 200 µl Buffer P1 (QIAGEN, Valencia, CA), 200 µl Buffer P2 (QIAGEN) were added, mixed thoroughly by inverting the sealed tube 4-6 times. Then the tubes were incubated at room temperature for 5 minutes. After incubating 200 µl Buffer P3 (QIAGEN) were added, inverted carefully 4-6 times and incubated on ice for 5 minutes. After 5 minutes the tubes got centrifuged at 13000 x g for 10 minutes. The supernatant was transferred properly in a new tube and 450 µl isopropanol were added, inverted 4-6 times, incubated on ice for 10 minutes and centrifuged at 13000 x g for 30 minutes at 4 °C. The supernatant was removed and 1 mL 70 % ethanol was added to the plasmid-DNA pellet and mixed properly. The last centrifugation step was performed at 13000 x g for 15 minutes at 4 °C. The supernatant was removed and the pellet was dried on a thermo block at 50 °C. Afterwards the white pellet was dissolved in 50 µl distilled water and put on a thermo block at 65 °C for better solving. The purified plasmid-DNA of each colony was analyzed by agarose gel electrophoresis. Plasmid-DNA of one colony, which showed intense bands, was purified via QIAGEN PCR Purification Kit, following the manufacturer's instructions and again analyzed by agarose gel electrophoresis.

### Preparation of large-scale cultures and induction of protein expression:

A single colony of *E. coli* BL21 cells containing pARMA2 for GST::*err1* expression was used for inoculating 4 mL LB/amp media and got incubated for 8 h at 37 °C with shaking at 160 rpm. The pre-culture was diluted 1:10 into 50 mL LB/amp media and grown at 37 °C with shaking at 160 rpm until it reached an OD600 of 1.676. The 50-mL-culture was diluted to an OD600 of 0.2 in a 250-mL-culture. Incubation at 37 °C with shaking was continued until the culture reached an OD600 of 0.752. At this point, the OD600 was optimal for induction which was initiated by adding IPTG (20 mg/mL) to a final concentration of 0.1 mM, and incubation was continued for additional 3 hours. The 250-mL-culture was transferred to a 50mL tube and centrifuged at 4000 rpm for 5 minutes at 4 °C. The supernatant was discarded, the cell pellet was solved in 2 mL PBS/Triton X-100 buffer and transferred to a 15mL tube and placed on ice.

### Extraction of the GST-err1 fusion proteins:

The cells were disrupted on ice with a sonicator in short bursts with following experimental settings: Cycle 50, 70 % energy, 30 seconds sonication and 30 seconds pause, repeated 4 times. Bacterial lysates were removed by centrifugation at 5000 rpm for 10 min and 0.5 mL of the clear supernatant (cell-free extract) were transferred to a reaction tube and stored at -80°C after adding 20 % (v/v) glycerol.

### Purification of the GST-err1 fusion proteins:

The GST fusion proteins in the cell-free extract were purified by affinity chromatography using glutathione immobilized to a matrix. Therefore, glutathione/sepharose columns (Amersham Bioscience, part of GE Healthcare, CT, US) with 1 mL column volume were used for purification and the following buffers were prepared:
- Binding buffer: PBS buffer, containing 140 mM NaCl, 2.6 mM KCl, 10 mM Na2HPO4, 1.8 mM KH2PO4, pH 7.3 and adding protease inhibitors 1 µM pepstatin A, 1 µM leupeptin, 0.5 µM PMSF right before usage.
- Elution buffer: 50 mM Tris-HCl, 10 mM glutathione, pH 8.0, adding protease inhibitors 1 µM pepstatin A, 1 µM leupeptin, 0.5 5 µM PMSF right before elution.

Before applying to the column, the cell-free extract was filtered through a 0.45 µm filter and the column was equilibrated by 10-fold column volumes of binding buffer and a flow rate of 1 mL/min was maintained. Then, cell-free extract was applied on the column with a flow rate of 0.5 mL/min, the eluate was collected and stored at 4 °C for analysis and the column was washed with 10-fold column volumes of binding buffer. The GST fusion proteins were eluted with 5-fold column volumes of elution buffer and collected as 0.5 mL aliquots in 10 1.5mL reaction tubes.

The concentration of each collected fraction was measured via NanoDrop at 280 nm and the fractions which showed the highest protein concentrations were united and divided equally to 4 reaction tubes. The aliquots were mixed with 20 % (v/v) glycerol and stored at -80 °C for analysis.

### Verification of purified expression products via SDS-PAGE:

The eluted expression products were verified and separated according to their size by SDS-PAGE at constant current of 12 mA (using the Mini-PROTEAN System, Bio-Rad) followed by PageBlue™ (Fermentas) protein staining according to a fast staining protocol for mini-gels according to the manufacturers instructions. Therefor, the following reagents were required: 5x Tris-glycine buffer containing SDS (pH 8.8), SDS-polyacrylamide gel containing 4-15 % Tris-HCl (BioRad, cat. No. 161-1104), and 5 x SDS loading dye, PageRuler™ Prestained Protein Ladder (10 - 170 kDa) (Fermentas), and PageBlue™ Protein Staining Solution (Fermentas) containing Coomassie Brilliant Blue G□250. On the gel, 45 µg (15 µl) purified GST-*err1* fusion protein and 10 µl protein ladder were applied. After staining, the gel was stored in distilled water at 4 °C for later reference.

### Enzyme assay measuring NADPH-decrease

Enzyme assay:
The following 1 mL assay was performed: 400 µM NADPH (in 10 mM sodium phosphate buffer, pH 7), and 25 µL purified GST::*err1* enzyme were mixed in an 1mL quartz cuvette and incubated at 30 °C for 1 min. Then adding D-erythrose to a final concentration of 10 mM started the reaction. The decrease of NADPH concentration was measured in a spectrophotometer at 340 nm. As a control, a blank was performed using water instead of D-erythrose. As a reference this assay was repeated using glyoxal, Larabinose, L-arabitol, D-xylose, xylitol, D-xylulose, and L-xylulose as substrates.

### HPLC analysis

### Enzyme assay:

The following 1 mL assays have been performed prior to HPLC analysis: Assay 1 comprised 4 mM NADPH (in 10 mM sodium phosphate buffer, pH 7), 25 µL purified GST::*err1* enzyme, 5 mM erythrose and was incubated at 25 °C for 2 hours. Assay 2 was performed according to assay 1, but was incubated at 37 °C for 2 h. Both assays were stopped by incubating at 85 °C for 10 min and afterwards were filtered using 0.2 µm filters.

As negative control assay 3 was performed like assay 1, but was immediately stopped by incubation at 85 °C for 10 min and assay 4 was performed according to assay 1, but sodium phosphate buffer instead of erythrose was used and it was immediately stopped at 85 °C for 10 min.

### HPLC analysis:

Analyses were performed using a Thermo Finnigan Surveyor HPLC instrument (Thermo Fisher Scientific, MA, US). All 10 µL samples were injected onto a Rezex RHM-Monosaccharide column (H+, 8 %, 150 x 7.8 mm; Phenomenex, CA, US). Water was used as the mobile phase and elution was followed at 85°C, applying a flow rate of 0.3 mL/min for 20 min. 1 mM D-Erythrose (Sigma) and erythritol (Sigma) were used as reference substances.

### Overexpression of err1

The native *T. reesei pki* (pyruvate kinase-encoding) and *bxl1* (beta-xylosidase-encoding, this enzyme cleaves the linkage between the two D-xylose units of which xylobiose consists of) promoters were used for overexpression of *err1.* This leads to a constitutive (*pki*) or an D-xylose inducible *(bxl1)* expression of *err1.* In both cases the *cbh2* (cellobiohydrolase II-encoding) terminator was applied.

The resulting recombinant *T. reesei* strain which converts D-xylose via erythrose to erythritol can be fed with D-xylose or can grow on hemicellulosic substrates and provide D-xylose by its own saprophytic activity.

The D-xylose inducible system has benefits because D-xylose is an indispensable substance (educt or metabolite) in this conversion anyway. Sometimes in nature, problems occur with systems which are self-inducible, e.g., because the organism's cell integrity becomes disturbed. In such a case, a constitutive system can prove advantageous.

Apart from the above promotors, other constitutive, e.g., *tef, gpd,* or inducible promoters, e.g., *cbh1, xyn1* can be applied as well.

### Results

The GLD1 protein from *Trichoderma reesei,* initially described as glycerol-dehydrogenase (GLD) by Liepins et al. (FEBS Journal 273 (2006), 4429-4235), has been found to exhibit erythrose reductase activity contrary to the earlier assumption, because it has the capability to convert erythrose to erythritol. The tests that show this finding are described in the following. Therefore, this enzyme was termed Erythrose reductase 1 (encoded by *err1*) by the inventors. Err1 was cloned and was subsequently used for production of erythritol in a recombinant *T. reesei* strain.
a) Determination of the enzymatic activity by analysis of the NADPH-decrease in an enzyme assay using GST::*err1* fusion proteins with different substrates:
According to Liepins et al. (2006) D-xylose was used as "negative" control and glyoxal as "positive" control because enzymatic activities on these substrates have been described.

| **Substrate** | **Absorbance decrease (mAbs/min)** |
|---|---|
| Glyoxal | -83,28 |
| D-xylose | -10,20 |
| Erythrose | -83,77 |

b) HPLC-Analyses of the enzymatic conversion of erythrose using heterologously expressed GST::*err1* fusion proteins gave clear indication that erythrose is reduced to erythritol.
c) The determination of Vmax and Km according to Liepins et al. (2006) gave clear indication that the preferred substrate for Err1 is erythrose and the reduction of it is not only the result of any star activity. Lipiens et al. referred to D-glyceraldehyde and methylglyoxal as substrates with good activity for GLD1, and they could detect no activity using glycerol (ND, no detection). The tests carried out by the inventors showed that erythrose has approximately 10-fold higher activity (see Table 1).

**Table 1**

| Substrate | Vₘₐₓ (nkat/mg) | Kₘ (mM) | Vₘₐₓ/Kₘ (/s M) |
|---|---|---|---|
| D-Glyceraldehyde | 150 | 0,9 | 6100 |
| Glyoxal | 375 | 2,4 | 5800 |
| Glycerol | ND | ND | |
| D-Xylose | 450 | 334 | 48 |
| Erythrose | 530 | 0,016 | 33125 |

## Claims

1. A method for the production of erythritol, wherein erythrose is reduced to erythritol with a NADPH-specific erythrose reductase, **characterized in that** an erythrose reductase from the group of saprophytes selected from the strains of *Hypocrea, Gibberella, Aspergillus* and *Penicillium* is used for reduction.

2. The method according to claim 1, **characterized in that** an erythrose reductase selected from the group of strains consisting of *Hypocrea jecorina* (*Trichoderma reesei*), *Gibberella zeae, Aspergillus niger, Aspergillus nidulans, Aspergillus oryzae* and *Penicillium chrysogenum* is used for reduction.

3. The method according to claim 1 or 2, **characterized in that** an erythrose reductase from *Trichoderma reesei* is used for reduction.

4. The method according to claim 3, **characterized in that** an erythrose reductase which is encoded by nucleic acid sequence SEQ ID NO:1 is used for reduction.

5. The method according to claim 3 or 4, **characterized in that** a protein having a sequence SEQ ID NO:2 is used for reduction.

6. The method according to any of claims 1 to 5, **characterized in that** it is carried out as a whole cell process.

7. The method according to claim 6, **characterized in that** a recombinant strain overexpressing the erythrose reductase is used.

8. The method according to claim 7, **characterized in that** overexpression of a erythrose reductase-coding gene of the recombinant strain is controlled by a promotor which is induced by xylose.


SEQUENCE LISTING

<110>  Annikki GmbH

<120>  Method for the production of erythritol

<130>  A 14124

<160>  2

<170>  PatentIn version 3.3

<210>  1
<211>  996
<212>  DNA
<213>  Hypocrea jecorina

<400>  1
atgtcttccg gaaggaccgt caccctcaac accggctaca agatccccca gatcggctac      60

ggcacctggc aggccgctcc cggcgaggtc ggcgctggtg tctttgaggc cctcaaggtt     120

ggctaccgcc acctcgacct ggccaaggtc tacggcaacc agaaggaggt tggtgagggc     180

atcaagaagg ctcttgctga ggtccccggc ctgaagcgcg aggatatttt catcacctcc     240

aagctgtgga acaactccca caagcccgag gacgtcgagc ccgctctcga cgacaccctg     300

gccgagcttg gcctcgacta ccttgacctc tacctcatcc actggcccgt tgcctttgct     360

cccggcgccg acctcttccc caagtccgag gacggctccg aggtgcagct caaccagaat     420

gtgtccattg tccagacctg gaaggccatg accgagctgc ccaagtccaa ggtccgctcc     480

gtcggtgtct ccaactttac cattgagcac ctcgacgccg tcatcgaggc caccggcgtc     540

gtccccgccg tcaaccagat cgagcgccac ccccgcctcc ccaaccagcc cctgatcgac     600

tactgcgcca agaagggcat catcatcacc gcctactccg cctttggcaa caacacaaag     660

ggcctgcccc tgctcgtcag ctccgacgag gtcaaggccg tcgccgacaa cctgtccaag     720

aagcagggca agaccgtcac tcccgcccag gtcatcctcg cctggtccca gattggtggc     780

cacaccgtca ttcccaagtc cgtcaccaag gcgcgcattg cggagaactt ccaggaggtt     840

gagctggatg acgaggccat tgctgcgctg aacaagttgg gcgagaagcc tcagcggttc     900

aacattcctt acacctacaa gcctaggtgg aacattaacc tgttcaacac cgaggaggag     960

aaggccgctg cccacactgc tgtcatcaag ctgtaa                               996

<210>  2
<211>  331
<212>  PRT
<213>  Hypocrea jecorina

<400>  2

Met Ser Ser Gly Arg Thr Val Thr Leu Asn Thr Gly Tyr Lys Ile Pro
1               5                   10                  15

Gln Ile Gly Tyr Gly Thr Trp Gln Ala Ala Pro Gly Glu Val Gly Ala
            20                  25                  30

Gly Val Phe Glu Ala Leu Lys Val Gly Tyr Arg His Leu Asp Leu Ala

35                  40                  45

Lys Val Tyr Gly Asn Gln Lys Glu Val Gly Glu Gly Ile Lys Lys Ala
    50                  55                  60

Leu Ala Glu Val Pro Gly Leu Lys Arg Glu Asp Ile Phe Ile Thr Ser
65                  70                  75                  80

Lys Leu Trp Asn Asn Ser His Lys Pro Glu Asp Val Glu Pro Ala Leu
                85                  90                  95

Asp Asp Thr Leu Ala Glu Leu Gly Leu Asp Tyr Leu Asp Leu Tyr Leu
            100                 105                 110

Ile His Trp Pro Val Ala Phe Ala Pro Gly Ala Asp Leu Phe Pro Lys
        115                 120                 125

Ser Glu Asp Gly Ser Glu Val Gln Leu Asn Gln Asn Val Ser Ile Val
    130                 135                 140

Gln Thr Trp Lys Ala Met Thr Glu Leu Pro Lys Ser Lys Val Arg Ser
145                 150                 155                 160

Val Gly Val Ser Asn Phe Thr Ile Glu His Leu Asp Ala Val Ile Glu
                165                 170                 175

Ala Thr Gly Val Val Pro Ala Val Asn Gln Ile Glu Arg His Pro Arg
            180                 185                 190

Leu Pro Asn Gln Pro Leu Ile Asp Tyr Cys Ala Lys Lys Gly Ile Ile
        195                 200                 205

Ile Thr Ala Tyr Ser Ala Phe Gly Asn Asn Thr Lys Gly Leu Pro Leu
    210                 215                 220

Leu Val Ser Ser Asp Glu Val Lys Ala Val Ala Asp Asn Leu Ser Lys
225                 230                 235                 240

Lys Gln Gly Lys Thr Val Thr Pro Ala Gln Val Ile Leu Ala Trp Ser
                245                 250                 255

Gln Ile Gly Gly His Thr Val Ile Pro Lys Ser Val Thr Lys Ala Arg
            260                 265                 270

Ile Ala Glu Asn Phe Gln Glu Val Glu Leu Asp Asp Glu Ala Ile Ala
        275                 280                 285

Ala Leu Asn Lys Leu Gly Glu Lys Pro Gln Arg Phe Asn Ile Pro Tyr
    290                 295                 300

Thr Tyr Lys Pro Arg Trp Asn Ile Asn Leu Phe Asn Thr Glu Glu Glu
305                 310                 315                 320

Lys Ala Ala Ala His Thr Ala Val Ile Lys Leu
                325                 330

SEQUENCE LISTING

<110>  Annikki GmbH

<120>  Method for the production of erythritol

<130>  A 14124

<140>  EP 10183799.5
<141>  2010-09-30

<160>  4

<170>  PatentIn version 3.3

<210>  1
<211>  996
<212>  DNA
<213>  Hypocrea jecorina

<400>  1
atgtcttccg gaaggaccgt caccctcaac accggctaca agatccccca gatcggctac      60

ggcacctggc aggccgctcc cggcgaggtc ggcgctggtg tctttgaggc cctcaaggtt     120

ggctaccgcc acctcgacct ggccaaggtc tacggcaacc agaaggaggt tggtgagggc     180

atcaagaagg ctcttgctga ggtccccggc ctgaagcgcg aggatatttt catcacctcc     240

aagctgtgga acaactccca caagcccgag gacgtcgagc ccgctctcga cgacaccctg     300

gccgagcttg gcctcgacta ccttgacctc tacctcatcc actggcccgt tgcctttgct     360

cccggcgccg acctcttccc caagtccgag gacggctccg aggtgcagct caaccagaat     420

gtgtccattg tccagacctg gaaggccatg accgagctgc ccaagtccaa ggtccgctcc     480

gtcggtgtct ccaactttac cattgagcac ctcgacgccg tcatcgaggc caccggcgtc     540

gtccccgccg tcaaccagat cgagcgccac ccccgcctcc ccaaccagcc cctgatcgac     600

tactgcgcca agaagggcat catcatcacc gcctactccg cctttggcaa caacacaaag     660

ggcctgcccc tgctcgtcag ctccgacgag gtcaaggccg tcgccgacaa cctgtccaag     720

aagcagggca agaccgtcac tcccgcccag gtcatcctcg cctggtccca gattggtggc     780

cacaccgtca ttcccaagtc cgtcaccaag gcgcgcattg cggagaactt ccaggaggtt     840

gagctggatg acgaggccat tgctgcgctg aacaagttgg gcgagaagcc tcagcggttc     900

aacattcctt acacctacaa gcctaggtgg aacattaacc tgttcaacac cgaggaggag     960

aaggccgctg cccacactgc tgtcatcaag ctgtaa                               996

<210>  2
<211>  331
<212>  PRT
<213>  Hypocrea jecorina

<400>  2

Met Ser Ser Gly Arg Thr Val Thr Leu Asn Thr Gly Tyr Lys Ile Pro
1               5                   10                  15

Gln Ile Gly Tyr Gly Thr Trp Gln Ala Ala Pro Gly Glu Val Gly Ala

20                  25                  30

Gly Val Phe Glu Ala Leu Lys Val Gly Tyr Arg His Leu Asp Leu Ala
        35                  40                  45

Lys Val Tyr Gly Asn Gln Lys Glu Val Gly Glu Gly Ile Lys Lys Ala
    50                  55                  60

Leu Ala Glu Val Pro Gly Leu Lys Arg Glu Asp Ile Phe Ile Thr Ser
65                  70                  75                  80

Lys Leu Trp Asn Asn Ser His Lys Pro Glu Asp Val Glu Pro Ala Leu
                85                  90                  95

Asp Asp Thr Leu Ala Glu Leu Gly Leu Asp Tyr Leu Asp Leu Tyr Leu
            100                 105                 110

Ile His Trp Pro Val Ala Phe Ala Pro Gly Ala Asp Leu Phe Pro Lys
        115                 120                 125

Ser Glu Asp Gly Ser Glu Val Gln Leu Asn Gln Asn Val Ser Ile Val
    130                 135                 140

Gln Thr Trp Lys Ala Met Thr Glu Leu Pro Lys Ser Lys Val Arg Ser
145                 150                 155                 160

Val Gly Val Ser Asn Phe Thr Ile Glu His Leu Asp Ala Val Ile Glu
                165                 170                 175

Ala Thr Gly Val Val Pro Ala Val Asn Gln Ile Glu Arg His Pro Arg
            180                 185                 190

Leu Pro Asn Gln Pro Leu Ile Asp Tyr Cys Ala Lys Lys Gly Ile Ile
        195                 200                 205

Ile Thr Ala Tyr Ser Ala Phe Gly Asn Asn Thr Lys Gly Leu Pro Leu
    210                 215                 220

Leu Val Ser Ser Asp Glu Val Lys Ala Val Ala Asp Asn Leu Ser Lys
225                 230                 235                 240

Lys Gln Gly Lys Thr Val Thr Pro Ala Gln Val Ile Leu Ala Trp Ser
                245                 250                 255

Gln Ile Gly Gly His Thr Val Ile Pro Lys Ser Val Thr Lys Ala Arg
            260                 265                 270

Ile Ala Glu Asn Phe Gln Glu Val Glu Leu Asp Asp Glu Ala Ile Ala
        275                 280                 285

Ala Leu Asn Lys Leu Gly Glu Lys Pro Gln Arg Phe Asn Ile Pro Tyr

290                     295                     300

Thr Tyr Lys Pro Arg Trp Asn Ile Asn Leu Phe Asn Thr Glu Glu Glu
305                 310                 315                 320

Lys Ala Ala Ala His Thr Ala Val Ile Lys Leu
                325                 330

<210>  3
<211>  28
<212>  DNA
<213>  Artificial

<220>
<223>  PCR primer err1_SalITf

<400>  3
atagtcgact atgtcttccg gaaggacc                                          28

<210>  4
<211>  33
<212>  DNA
<213>  Artificial

<220>
<223>  PCR primer err1_NotIr

<400>  4
tatgcggccg cttacagctt gatgacagca gtg                                    33